# EUROPEAN PATENT APPLICATION

(11) **EP 3 504 962 A1**
(43) Date of publication of application: **03.07.2019**
(21) Application number: 17843468.4
(22) Date of filing: 17.08.2017
(51) Int. Cl.: A01H 5/00, A01G 2/30, C12N 15/09, C12N 7/01

(54) **PLANT'S CHARACTER REGULATION METHOD**

(30) Priority: 25.08.2016 JP 2016164729
(71) Applicant: National University Corporation Nagoya University, Nagoya-shi, Aichi 464-8601 (JP)
(72) Inventor: NOTAGUCHI, Michitaka, Nagoya-shi, Aichi 464-8601 (JP); NIWA, Masaki, Nagoya-shi, Aichi 464-8601 (JP); OTAGAKI, Shungo, Nagoya-shi, Aichi 464-8601 (JP)
(74) Representative: Gevers & Orès
(86) International application number: PCT/JP2017/029502
(87) International publication number: WO 2018/037986

(57) **Abstract**

Provided are more efficient conditions for allowing a characteristic regulator to function, by using an inter-family-graftable plant of a specific family, such as Solanaceae, as a medium, in a plant belonging to a different family from that of the medium. A plant virus for producing a characteristic regulator is selected as the delivery medium, and is delivered to a different-family plant through an inter-family-graftable plant of a specific family, such as Solanaceae.

## Description

### Technical Field

The present invention relates to a plant characteristic-regulating method and a useful medium for the method.

### Background Art

Grafting is a technology for physically joining two or more plants into a single plant. Grafting is commonly carried out on gymnosperms and angiosperms, and is widely used horticulturally and agriculturally. In general, grafting is a technology consisting of preparing rootstock composing the root portion and budwood serving as the aboveground portion, and is used to produce a plant body that demonstrates both of the superior capabilities of each component. The objectives of and techniques for grafting are diverse. For example, there are many cases in which bud sports or new varieties of fruit trees in general are cloned and propagated by grafting. In addition, vegetables including members of the Solanaceae and Cucurbitaceae families have been made to acquire disease resistance and achieve improvements in fruit quality and productivity by utilizing useful root systems produced through grafting.

Grafting is not a method that can be applied in all sorts of combinations, and is considered to be established more easily the closer the phylogenetic relationship. Grafting is considered to be established when at least two plant bodies reach a thriving state through the grafted surface. In general, grafting is increasingly less likely to become established when carried out between plants of the same species, plants of the same genus and plants of the same family in that order, and although grafting between specific different-family plants has been reported as an exception thereto (NPL 1 and 2), grafting between different-family plants does not become established in general.

Amidst these circumstances, the inventors of the present invention reported that plants of a specific family such as Solanaceae are able to be grafted with a diverse range of different-family plants (PTL 1). According to this technology, the use of a plant of a specific family such as Solanaceae makes it possible to a certain degree to deliver trace amounts of useful substances to a different-family plant. However, in the case of grafting between different-family plants, the efficiency of material transport is considered to be lower in comparison with ordinary grafting demonstrating a high degree of affinity between closely related plant species. In the case of ordinary grafting between closely related plant species, parenchyma that develops at the grafted site differentiates, the tissue of both of the mutually grafted plants is reconstructed to the original tissue, and phloem that transports nutrients and xylem that transports water are connected. On the other hand, in the case of the inter-family grafting method reported in PTL 1, although the phenomenon is the same through differentiation of parenchyma at the grafted site, subsequent tissue reconstruction is not established to the degree it is achieved in the case of grafting between closely related plant species, and although the development of phloem and xylem, which are important as material transport pathways, is partially observed, the direction of the formation thereof is random, and material transport is not as adequately achieved in comparison with grafting between closely related plant species. Consequently, a technology is desired for delivering amounts of substances sufficient for characteristic regulation.

### Citation List

### Patent Literature

PTL 1: WO 2016/060189

### Non-patent Literature

NPL 1: Simon, S.V., Jahrb. wiss. Bot., 1930.72, 137-160.
NPL 2: Nickell, L.G., Science, 1948. 108. 389.

### Summary of Invention

### Technical Problem

During the course of conducting research on grafting between a plant of a specific family such as Solanaceae and a different-family plant, the inventors of the present invention found that, in the case of allowing a characteristic regulator to function by delivering the regulator to a different-family plant, there are considerable differences in the efficiency thereof depending on the conditions (such as the type and delivery method of the characteristic regulator).

Therefore, an object of the present invention is to provide more efficient conditions for allowing a characteristic regulator to function, by using an inter-family-graftable plant of a specific family, such as Solanaceae, as a medium, in a plant belonging to a different family from that of the medium. A plant virus for producing a characteristic regulator is selected as the delivery medium, and is delivered to a different-family plant through an inter-family-graftable plant of a specific family, such as Solanaceae.

### Solution to Problem

As a result of conducting extensive studies on various choices for achieving the above-mentioned object with the foregoing in view, the inventors of the present invention surprisingly found that, a characteristic regulator can be made to function more efficiently in a different-family plant by delivering a delivery agent in the form of a plant virus for producing a characteristic regulator to a different-family plant through an inter-family-graftable plant of a specific family such as Solanaceae.

In PTL 1, since xylem elements and phloem elements are formed in random directions at the grafted site, and the orientation of phloem elements and xylem elements within the parenchyma thereof is disorderly, the efficiency of material transport has been suggested to be lower in comparison with ordinary grafting between closely related plant species.

Moreover, the material transport indicated in PTL 1 was limited to that simply relating to substances capable of being transport passively. Although plasmodesma responsible for symplastic material transport between cells is indicated to be formed, there is no description regarding the functionality thereof. In general, viruses interact with constituents of plasmodesma when migrating between cells or are able to migrate by altering their structures. Since viruses require such active migration, the structure used by viruses during migration was not thought to be formed functionally in plasmodesma newly formed on the inter-family grafted surface.

For this reason, since migration of an amount of virus sufficient for characteristic regulation was able to be detected, the formation of a functional structure enabling such active migration in the present invention was extremely surprising.

Namely, the present invention includes the aspects indicated below.
1. A characteristic regulation delivery medium for delivering a plant virus for producing a characteristic regulator to a different-family plant, the delivery medium including a partial structure of a plant belonging to the family Solanaceae, Brassicaceae, Lamiaceae, or Orobanchaceae.
2. The delivery medium described in 1, which is a delivery medium for delivery to a different-order plant.
3. The delivery medium described in 1 or 2, wherein the partial structure is a partial structure of a plant belonging to the family Solanaceae.
4. The delivery medium described in any one of 1 to 3, wherein the partial structure is a partial structure of a plant belonging to the genus Nicotiana.
5. The delivery medium described in any one of 1 to 4, wherein the characteristic regulator is at least one member selected from the group consisting of RNA and proteins.
6. The delivery medium described in any one of 1 to 5, wherein the delivery medium contains a plant virus for producing a characteristic regulator.
7. A partial structure including the delivery medium described in any one of 1 to 6 grafted with a different-family plant directly or through another plant.
8. A plant body containing the partial structure described in 7.
9. A plant body characteristic-regulating method including delivering a plant virus for producing a characteristic regulator to the plant body described in 8 through the delivery medium described in any one of 1 to 6 contained in the plant body.
10. A method for producing a characteristic-regulated plant body, the method including delivering a plant virus for producing a characteristic regulator to the plant body described in 8 through the delivery medium described in any one of 1 to 6 contained in the plant body.
11. The characteristic-regulating method described in 9, the method including grafting the delivery medium described in 6 with a different-family plant directly or through another plant.
12. The production method described in 10, the method including grafting the delivery medium described in 6 with a different-family plant directly or through another plant.
13. A seed obtained from the plant body described in 8 or an offspring plant body of the plant body described in 8.
14. A seed group of a characteristic-regulated plant body obtained from the plant body described in 8 or an offspring plant body of the plant body described in 8.
15. A method for producing a seed of a characteristic-regulated plant body, the method including obtaining a seed from the plant body described in 8 or an offspring plant body of the plant body described in 8.

### Advantageous Effects of Invention

According to the present invention, a characteristic regulator can be allowed to function more efficiently in a different-family plant by delivering a plant virus for producing the characteristic regulator to the different-family plant using an inter-family-graftable plant of a specific family such as Solanaceae as a medium. As a result, various types of plant characteristics such as growth, morphology, flowering or fruiting can be controlled, or microorganisms such as bacteria or viruses as well as animals such as insects that use a plant body as a host can be attracted or controlled over the current generation and subsequent generations, depending on the type of characteristic regulator (such as protein or RNA).

### Brief Description of Drawings

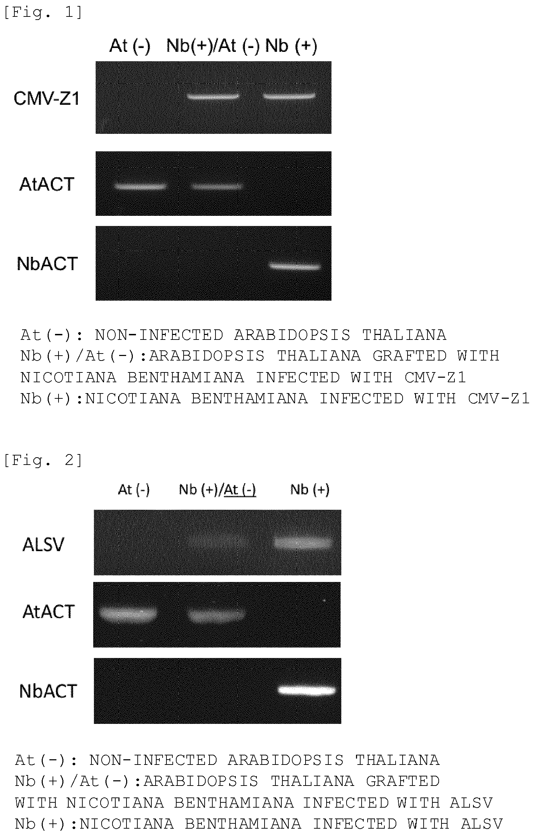

Fig. 1 indicates stained images of electrophoresed gel obtained in Example 1-4; above the images, At(-) indicates a sample of a negative control (non-virus-infected Arabidopsis thaliana), Nb(+)/At(-) indicates a sample obtained in Example 1-3, and Nb(+) indicates a sample of a positive control (virus-infected Nicotiana benthamiana); and on the left side of the images, CMV-Z1 indicates the case of having carried out PCR using a primer set for detecting CMV, AtACT indicates the case of having carried out PCR using a primer set specific for Arabidopsis thaliana actin gene, and NbACT indicates the case of having carried out PCR using a primer set specific for Nicotiana benthamiana actin gene.
Fig. 2 indicates stained images of electrophoresed gel obtained in Example 2-4; above the images, At(-)indicates a sample of a negative control (non-virus-infected Arabidopsis thaliana), Nb(+)/At(-) indicates a sample obtained in Example 2-3, and Nb(+) indicates a sample of a positive control (virus-infected Nicotiana benthamiana); and on the left side of the images, ALSV indicates the case of having carried out PCR using a primer set for detecting ALSV, AtACT indicates the case of having carried out PCR using a primer set specific for Arabidopsis thaliana actin gene, and NbACT indicates the case of having carried out PCR using a primer set specific for Nicotiana benthamiana actin gene.

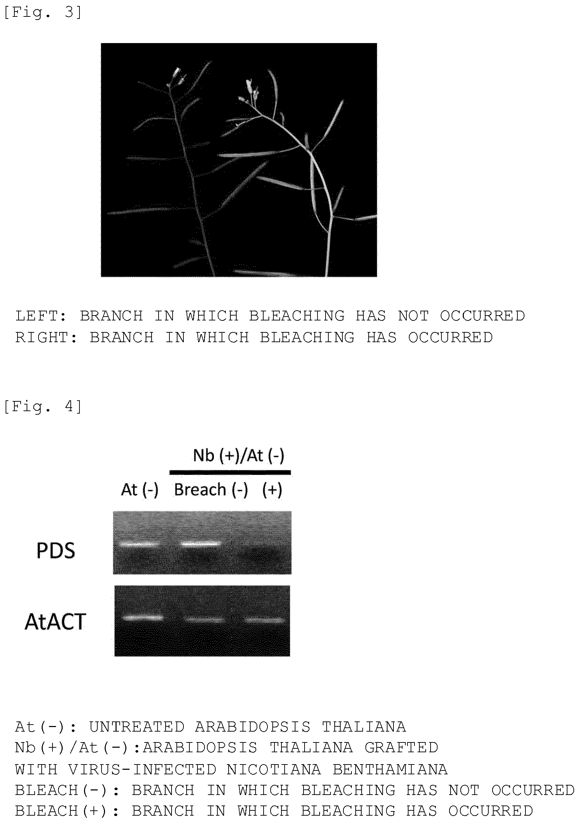

Fig. 3 indicates a photograph of plant bodies obtained in Example 4-3; and the left side of the photograph indicates a branch of Arabidopsis thaliana in which bleaching did not occurred, while the right side of the photograph indicates a branch of Arabidopsis thaliana in which bleaching occurred.
Fig. 4 indicates stained images of electrophoresed gel obtained in Example 4-4; above the images, At(-) indicates a sample of a negative control (non-virus-infected Arabidopsis thaliana), Nb(+)/At(-) indicates a sample obtained in Example 4-3, and within the Nb(+)/At(-), Bleach(-) indicates a sample of a branch in which bleaching did not occurred, while Bleach(+) indicates a sample of a branch in which bleaching occurred; and on the left side of the images, PDS indicates the case of having carried out PCR using a primer set specific for PDS gene, and AtACT indicates the case of having carried out PCR using a primer set specific for Arabidopsis thaliana actin gene.

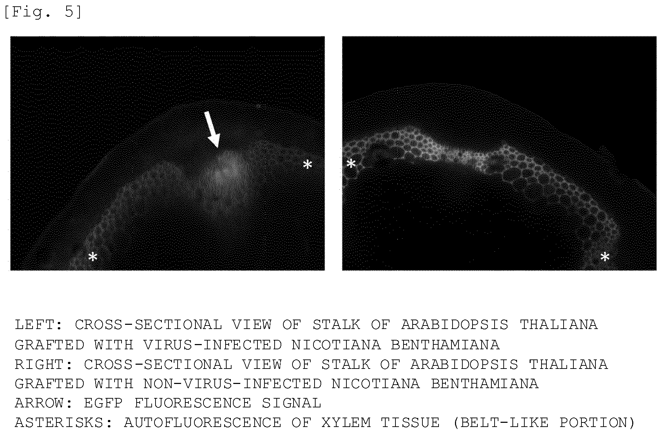

Fig. 5 indicates fluorescent micrographs depicting cross-sections of plant bodies obtained in Example 5-3; the micrograph on the left indicates a transverse cross-section of the stem of a sample obtained in Example 5-3, while the micrograph on the right indicates a transverse cross-section of the stem of a sample of a negative control (Arabidopsis thaliana grafted with non-virus-infected Nicotiana benthamiana); the arrow indicates EGFP fluorescence, while the belt-like portion situated between the asterisks indicates autofluorescence generated by xylem; and the micrograph on the left indicates a branch of Arabidopsis thaliana in which bleaching did not occurred, while the micrograph on the right indicates a branch of Arabidopsis thaliana in which bleaching occurred.

### Description of Embodiments

In the present description, the expressions "comprising," "containing," and "including" include the concepts of "comprising," "containing", "including", "consisting essentially of" and "consisting of".

### 1. Delivery Medium

In one aspect thereof, the present invention relates to a characteristic regulation delivery medium (to also be indicated as the "delivery medium of the present invention" in the present description) for delivering a plant virus for producing a characteristic regulator to a different-family plant, the delivery medium including a partial structure of a plant belonging to the family Solanaceae, Brassicaceae, Lamiaceae, or Orobanchaceae (to also be indicated as the "inter-family-graftable plant" in the present description). The following provides an explanation thereof.

Although examples of inter-family-graftable plants include plants of the families Solanaceae, Brassicaceae, Lamiaceae and Orobanchaceae, plants of the family Solanaceae are preferable from the viewpoint of not only enabling grafts with different-family plants to be established more efficiently, but also being able to deliver plant viruses for producing characteristic regulators more efficiently.

Examples of plants belonging to the family Solanaceae include, but are not particularly limited to, plants belonging to the genus Nicotiana, Anthocercis, Anthotroche, Crenidium, Cyphanthera, Duboisia, Grammosolen, Symonanthus, Petunia, Benthamiella, Bouchetia, Brunfelsia, Combera, Fabiana, Hunzikeria, Leptoglossis, Nierembergia, Pantacantha, Calibrachoa, Plowmania, Capsicum, Lycianthes, Solanum, Jaltomata, Datura, Brugmansia, Physalis, Physaliastrum, Tubocapsicum, Scopolia, Hyoscyamus, Atropa, Mandragora, Lycium or Calibrachoa. Among these, from the viewpoints of enabling grafts with different-family plants to be established more efficiently while also enabling plant viruses for producing characteristic regulators to be delivered more efficiently, plants belonging to the Nicotiana, Anthocercis, Anthotroche, Crenidium, Cyphanthera, Duboisia, Grammosolen, Symonanthus, Petunia, Benthamiella, Bouchetia, Brunfelsia, Combera, Fabiana, Hunzikeria, Leptoglossis, Nierembergia, Pantacantha, Calibrachoa, Plowmania, Capsicum, Lycianthes, Solanum and Jaltomata are preferable, plants belonging to the Nicotiana, Petunia, Capsicum and Solanum are more preferable, and plants belonging to the genus Nicotiana are even more preferable.

Examples of plants belonging to the genus Nicotiana include, but are not particularly limited to, Nicotiana benthamiana, Nicotiana tabacum, Nicotiana umbratica, Nicotiana rustica, Nicotiana acuminata, Nicotiana alata, Nicotiana attenuata, Nicotiana clevelandii, Nicotiana excelsior, Nicotiana forgetiana, Nicotiana glauca, Nicotiana glutinosa, Nicotiana langsdorffii, Nicotiana longiflora, Nicotiana obtusifolia, Nicotiana paniculata, Nicotiana plumbagifolia, Nicotiana quadrivalvis, Nicotiana repanda, Nicotiana suaveolens, Nicotiana sylvestris and Nicotiana tomentosa. Among these, from the viewpoints of enabling grafts with different-family plants to be established more efficiently while also enabling plant viruses for producing characteristic regulators to be delivered more efficiently, Nicotiana benthamiana, Nicotiana tabacum, Nicotiana umbratica and Nicotiana rustica are preferable, and Nicotiana benthamiana is more preferable.

Examples of plants belonging to the genus Petunia include, but are not particularly limited to, Petunia × atkinsiana (petunia), Petunia alpicola, Petunia axillaris, Petunia bajeensis, Petunia bonjardinensis, Petunia exserta, Petunia guarapuavensis, Petunia inflata, Petunia integrifolia, Petunia interior, Petunia ledifolia, Petunia littoralis, Petunia mantiqueirensis, Petunia occidentalis, Petunia patagonica, Petunia reitzii, Petunia riograndensis, Petunia saxicola, Petunia scheideana and Petunia villadiana. Among these, from the viewpoints of enabling grafts with different-family plants to be established more efficiently while also enabling plant viruses for producing characteristic regulators to be delivered more efficiently, Petunia × atkinsiana is preferable.

Examples of plants belonging to the genus Capsicum include, but are not particularly limited to, Capsicum annuum L. (such as 'Grossum' (green pepper), 'Abbreviatum', 'Acuminoum', 'Cerasiforme', 'Conoides', 'Fasciculatum', 'Longum', 'Nigrym', or 'Parvo-acuminatum'), Capsicum baccatum, Capsicum cardenasii, Capsicum Chinese Jacq. Heser & Smith, Capsicum frutescens L. and Capsicum pubescens Ruiz & Pav. Among these, from the viewpoints of enabling grafts with different-family plants to be established more efficiently while also enabling plant viruses for producing characteristic regulators to be delivered more efficiently, Capsicum annuum L. is preferable, and Capsicum annuum L. 'Grossum' (green pepper) is more preferable.

Examples of plants belonging to the genus Solanum include, but are not particularly limited to, Solanum lycopersicum L. (tomato), Solanum melongena L. (eggplant), Solanum tuberosum L., Solanum acaule Bitt., Solanum aethiopicum L., Solanum betaceum Cav., Solanum jasminoides Paxt., Solanum mammosum L., Solanum muricatum Aiton, Solanum nigrum L., Solanum pseudocapsicum L. and Solanum ptychanthum Dunal. Among these, from the viewpoints of enabling grafts with different-family plants to be established more efficiently while also enabling plant viruses for producing characteristic regulators to be delivered more efficiently, Solanum lycopersicum L. (tomato) and Solanum melongena L. (eggplant) are preferable.

Examples of plants belonging to the family Brassicaceae include, but are not particularly limited to, plants belonging to the genus Arabidopsis, Brassica, Capsella, Cardamine, Aethionema, Camelina, Armoracia, Barbarea, Nasturtium, Rorippa, Lepidium, Coronopus, Descurainia, Alyssum, Aurinia, Lobularia, Sisymbrium, Diplotaxis, Arugula, Raphanus, Hirschfeldia, Sinapis, Rapistrum, Orychophragmus, Isatis, Eutrema, Thlaspi, Arabis, Aubrietia, Draba, Macropodium, Noccaea, Iberis, Cochlearia, Malcolmia, Matthiola, Hesperis, Chorispora or Lunaria. Among these, from the viewpoints of enabling grafts with different-family plants to be established more efficiently while also enabling plant viruses for producing characteristic regulators to be delivered more efficiently, plants belonging to the genii Arabidopsis, Camelina, Brassica, Diplotaxis, Arugula, Raphanus, Hirschfeldia, Sinapis, Rapistrum, Orychophragmus, Capsella, Cardamine, Armoracia, Barbarea, Nasturtium and Rorippa are preferable, plants belonging to the genii Arabidopsis, Brassica, Capsella and Cardamine are more preferable, plants belonging to the genii Arabidopsis and Brassica are even more preferable, and plants belonging to the genus Arabidopsis are even still more preferable.

Examples of plants belonging to the genus Arabidopsis include, but are not particularly limited to, Arabidopsis thaliana (thale cress), Arabidopsis arenicola, Arabidopsis arenosa, Arabidopsis cabennensis, Arabidopsis croatica, Arabidopsis halleri, Arabidopsis lyrata, Arabidopsis neglecta, Arabidopsis pedemontana and Arabidopsis suecica. Among these, from the viewpoints of enabling grafts with different-family plants to be established more efficiently while also enabling plant viruses for producing characteristic regulators to be delivered more efficiently, Arabidopsis thaliana (thale cress) is preferable.

Examples of plants belonging to the genus Brassica include, but are not particularly limited to, Brassica Oleracea (such as broccoli, cauliflower or cabbage), Brassica Napus (such as rapeseed), Brassica Barrelieri, Brassica carinata, Brassica elongata, Brassica fruticulosa, Brassica juncea, Brassica narinosa, Brassica nigra, Brassica nipposinica, Brassica rapa, Brassica rupestris and Brassica Tournefortii. Among these, from the viewpoints of enabling grafts with different-family plants to be established more efficiently while also enabling plant viruses for producing characteristic regulators to be delivered more efficiently, Brassica Oleracea and Brassica Napus are preferable, Brassica Oleracea is more preferable, and broccoli is even more preferable.

Examples of plants belonging to the genus Capsella include, but are not particularly limited to, Capsella rubella (pink shepherd's-purse), Capsella abscissa, Capsella andreana, Capsella australis, Capsella austriaca, Capsella bursa-pastoris, Capsella divaricata, Capsella draboides, Capsella gracilis, Capsella grandiflora, Capsella humistrata, Capsella hybrida, Capsella hyrcana, Capsella integrifolia, Capsella lycia, Capsella mexicana, Capsella orientalis, Capsella pillosula, Capsella pubens, Capsella puberula, Capsella schaffneri, Capsella stellata, Capsella tasmanica, Capsella thomsoni, Capsella thracica, Capsella viguieri and Capsella villosula. Among these, from the viewpoints of enabling grafts with different-family plants to be established more efficiently while also enabling plant viruses for producing characteristic regulators to be delivered more efficiently, Capsella rubella (pink shepherd's-purse) is preferable.

Examples of plants belonging to the genus Cardamine include, but are not particularly limited to, Cardamine hirsuta (bitter cress), Cardamine anemonoides, Cardamine appendiculata, Cardamine arakiana, Cardamine dentipetala, Cardamine dentipetala var. longifructa, Cardamine fallax, Cardamine impatiens, Cardamine kiusiana, Cardamine leucantha, Cardamine lyrata, Cardamine niigatensis, Cardamine nipponica, Cardamine pratensis, Cardamine regeliana, Cardamine schinziana, Cardamine scutata, Cardamine tanakae, Cardamine torrentis and Cardamine valida. Among these, from the viewpoints of enabling grafts with different-family plants to be established more efficiently while also enabling plant viruses for producing characteristic regulators to be delivered more efficiently, Cardamine hirsuta (bitter cress) is preferable.

Examples of plants belonging to the family Lamiaceae include, but are not particularly limited to, plants belonging to the genus Perilla, Lavandula, Callicarpa, Vitex, Tectona, Premna, Ajuga, Clerodendrum, Caryopteris, Amethystea, Teucrium, Keiskea, Elsholtzia, Mosla, Agastache, Nepeta, Origanum, Mentha, Dracocephalum, Glechoma, Hyssopus, Prunella, Lycopus, Meehania, Melissa, Monarda, Salvia, Satureja, Rosmarinus, Thymus, Clinopodium, Rabdosia, Hyptis, Ocimum, Scutellaria, Stachys, Perillula, Lamium, Galeopsis, Chelonopsis, Pogostemon, Leucosceptrum, Leonurus, Loxocalyx, Leucas or Marrubium. Among these, from the viewpoints of enabling grafts with different-family plants to be established more efficiently while also enabling plant viruses for producing characteristic regulators to be delivered more efficiently, plants belonging to the genii Perilla, Lavandula, Keiskea, Elsholtzia, Mosla, Agastache, Nepeta, Origanum, Mentha, Dracocephalum, Glechoma, Hyssopus, Prunella, Lycopus, Meehania, Melissa, Monarda, Salvia, Satureja, Rosmarinus, Thymus, Clinopodium, Rabdosia, Hyptis and Ocimum are preferable, plants belonging to the genii Perilla and Lavandula are more preferable, and plants belonging to the genus Perilla are even more preferable.

Examples of plants belonging to the genus Perilla include, but are not particular limited to, Perilla frutescens (such as *shiso* plant or perilla frutescens). Among these, from the viewpoints of enabling grafts with different-family plants to be established more efficiently while also enabling plant viruses for producing characteristic regulators to be delivered more efficiently, Perilla frutescens is preferable and *shiso* is more preferable.

Examples of plants belonging to the genus Lavandula include, but are not particularly limited to, Lavandula angustifolia (lavender), Lavandula latifolia, Lavandula stoechas, Lavandula multifida and Lavandula × intermedia. Among these, from the viewpoints of enabling grafts with different-family plants to be established more efficiently while also enabling plant viruses for producing characteristic regulators to be delivered more efficiently, Lavandula angustifolia (lavender) is preferable.

Examples of plants belonging to the family Orobanchaceae include, but are not particularly limited to, plants belonging to the genus Phtheirospermum, Castilleja, Orthocarpus, Agalinis, Aureolaria, Esterhazya, Seymeria, Lamourouxia, Cordylanthus, Tyriphysaria, Aeginetia, Boschniakia, Cistanche, Orobanche, Phacellanthus, Euphrasia, Lathraea, Melampyrum, Monochasma, Parentucellia, Pedicularis, Siphonostegia or Striga. Among these, from the viewpoints of enabling grafts with different-family plants to be established more efficiently while also enabling plant viruses for producing characteristic regulators to be delivered more efficiently, plants belonging to the genii Phtheirospermum, Pedicularis, Castilleja, Orthocarpus, Agalinis, Aureolaria, Esterhazya, Seymeria, Lamourouxia, Cordylanthus and Triphysaria are preferable, and plants belonging to the genus Phtheirospermum are more preferable.

Examples of plants belonging to the genus Phtheirospermum include, but are not particularly limited to, Phtheirospermum japonicum, Phtheirospermum glandulosum, Phtheirospermum muliense, Phtheirospermum parishii and Phtheirospermum tenuisectum. Among these, from the viewpoints of enabling grafts with different-family plants to be established more efficiently while also enabling plant viruses for producing characteristic regulators to be delivered more efficiently, Phtheirospermum japonicum is preferable.

The above-mentioned "partial structure" of an inter-family-graftable plant refers to a structure composed of an arbitrary portion of the entire inter-family-graftable plant, and there are no particular limitations thereon provided it is a graftable structure. Examples thereof include a structure composed of all or a portion of an underground portion, a structure composed of all or a portion of an underground portion and all or a portion of an aboveground portion, and a structure composed of all or a portion of an aboveground portion. Furthermore, in the present description, there are no particular limitations on the underground portion provided it is a structure capable of existing while buried underground, and examples thereof include roots and rhizomes. In addition, in the present description, there are no particular limitations on the aboveground portion provided it is a structure capable of existing while exposed above ground, and examples thereof include stalks, trunks, branches, petioles, leaves, buds and flowers.

There are no particular limitations on the delivery medium of the present invention provided it includes a partial structure of an inter-family-graftable plant, and may be composed only of one or a plurality of partial structures of an inter-family-graftable plant or may be composed of the entire structure of an inter-family-graftable plant. In the case of containing two or more types of partial structures, each partial structure is normally in a grafted state.

There are no particular limitations on the delivery medium of the present invention provided it is a structure that can be grafted with a different-family plant. Examples thereof include a structure composed of all or a portion of an underground portion, a structure composed of all or a portion of an underground portion and all or a portion of an aboveground portion, and a structure composed of all or a portion of an aboveground portion.

There are no particular limitations on the form of the delivery medium of the present invention provided it is an inter-family-graftable form. From the viewpoint of enabling more efficient inter-family grafting, the delivery medium of the present invention preferably has a surface suitable for grafting in a portion thereof. Examples of surfaces suitable for grafting include cross-sectional surfaces that achieve favorable contact as necessary, such as various types of known forms, including a flat shape, V-shaped indentation, or protruding projection.

As explained in the section entitled, "3. Characteristic-Regulating Method", to be subsequently described, the delivery medium of the present invention can be used by grafting with a different-family plant (or in a state of being grafted with a different-family plant), and can be used to regulate a characteristic by delivering a plant virus for producing a characteristic regulator to the different-family plant through the delivery medium.

From the viewpoint of this application, the delivery medium of the present invention preferably contains parenchyma containing tissue such as procambium in which cell activity, such as cell division, is active. This is because parenchyma is thought to be able to mediate favorable grafting. Parenchyma refers to plant tissue composed of parenchymal cells. Examples of parenchymal tissue include the cortex and pith of stalks and roots, leaf palisade tissue and spongy tissue, xylem parenchymal tissue and phloem parenchymal tissue of vascular bundles, fruit pulp, tubers, tuberous roots, and other storage tissue.

Furthermore, in the present invention, grafting can be carried out in accordance or in compliance with known methods. In the present invention, grafting can be typically carried out by a method including cutting a plant at its arbitrary site (such as an arbitrary site on a stalk) to obtain two or more partial structures for grafting (such as stock, intermediate rootstock or budwood) (Step 1), and contacting the corresponding cross-sectional surfaces of the partial structures for grafting, followed by cultivation (Step 2).

In Step 1, the cross-sectional surfaces of the partial structures for grafting are preferably processed into a form that increases the contact area between the cross-sectional surfaces. For example, a notch measuring about 1 cm to 2 cm is made in the center of one of the cross-sectional surfaces, while the other cross-sectional surface is cut to have a V-shape. This processing can be carried out using, for example, a single-edged razor.

In Step 2, there are no particular limitations on the form of contact between the cross-sectional surfaces. Contact is preferably carried out gently so as not to damage the partial structures for grafting.

In Step 2, the contact site is preferably immobilized so as to prevent the contact site from moving as much as possible following grafting. Immobilization can be carried out by, for example, wrapping the contact site with a sheet-like material such as Parafilm.

During the initial stage of the cultivation of Step 2 (such as from 3 days to 14 days after contact), the entire partial structure for grafting located above ground (such as budwood or intermediate rootstock) is preferably maintained in a humidified state. Humidification can be carried out by, for example, covering the entire partial structure for grafting located above ground with a plastic bag sprayed with water.

In the case of having carried out humidification treatment during the initial stage of cultivation in Step 2, cultivation in the later stage (for example, about 30 days since contact) is preferably carried out in the absence of humidification treatment.

In the case of grafting three partial structures for grafting, two of the partial structures for grafting are first grafted according to the above-mentioned Steps 1 and 2, and the partial structure for grafting obtained from the resulting plant body is preferably grafted with the third partial structure for grafting. Grafting is preferably carried out using a method in compliance therewith in the case of grafting four or more partial structures for grafting as well.

There are no particular limitations on the "different-family plant" grafted with the delivery medium of the present invention provided it is a plant belonging to a different family from that of the delivery medium of the present invention. As indicated in PTL 1, the delivery medium of the present invention is able to establish a graft with a wide range of plants including pteridophytes, gymnosperms, angiosperm magnolids, monocots and eudicots (rosids I, rosids II, asterids I, asterids II and other outgroups thereof). Specific examples of different-family plants include plants belonging to the family Malvaceae, Brassicaceae, Asteraceae, Salicaceae, Ranunculaceae, Lauraceae, Chloranthaceae, Saururaceae, Araceae, Lamiaceae, Violaceae, Umbelliferae, Buxaceae, Ericaceae, Polygonaceae, Amaranthaceae, Convolvulaceae, Rosaceae, Santalaceae, Capparaceae, Geraniaceae, Vitaceae, Fagaceae, Caprifoliaceae, Dipsacaceae, Leguminosae, Rutaceae, Sapindaceae, Proteaceae, Saxifragaceae, Apocynaceae, Gentianaceae, Dryopteridaceae, Cupressaceae, Cucurbitaceae, Solanaceae, Pedaliaceae, Plantaginaceae, Orobanchaceae, Linderniaceae, Capparidaceae, Calyceraceae, Goodeniaceae, Menyanthaceae, Stylidiaceae, Polygalaceae, Surianaceae, Muntingiaceae, Cytinaceae, Dipterocarpaceae, Sarcolaenaceae, Cistaceae, Bixaceae, Sphaerosepalaceae, Tetramelaceae, Begoniaceae, Datiscaceae, Berberidaceae, Menispermaceae, Lythraceae, Astilbe, Scrophulariaceae, Mazaceae, Phrymaceae, Paulowniaceae, Piperaceae, Didymellaceae, Hypodematiaceae, Lomariopsidaceae, Nephrolepidaceae, Tectariaceae, Oleandraceae, Polypodiaceae, Davalliaceae, Hypodematiaceae, Lomariopsidaceae, Nephrolepidaceae, Tectariaceae, Oleandraceae, Polypodiaceae or Davalliaceae.

In the case where the delivery medium of the present invention comprises a partial structure of a plant belonging to the family Solanaceae, genus Nicotiana, examples of different-family plants grafted therewith include 58 families and 620 species of dicotyledons, 20 families and 213 species of monocotyledons and 16 species of pteridophytes that are preferably edible plants.

Among the above-mentioned food plants, examples of dicotyledons include 68 species of legumes such as soybeans, *adzuki* beans, green peas or black-eyed peas, 57 species of cucurbits such as cucumbers, melons, watermelons, or pumpkins, 63 species of solanaceous plants such as tobacco, eggplants, tomatoes or green peppers, 57 species of asteraceae plants such as crown daisies, butterbur, burdock or lettuce, 24 species of umbelliferous plants such as carrots, parsley, Japanese honewort or celery, 23 species of polygonaceous plants such as sorrel, knotweed, rhubarb or buckwheat, and 44 species of amaranthaceous plants such as spinach, saltwort, Swiss chard or beets.

In addition, among the above-mentioned edible plants, examples of monocotyledons include 50 species of liliaceous plants, 22 species of araceous plants, 26 species of dioscoreaceous plants and 40 species of gramineous plants. Additional examples include woody plants such as citrus family plants or palm family plants as well as 31 families and 149 species of spices.

In the case where the delivery medium of the present invention comprises a partial structure of a plant belonging to the genus Petunia, Capsicum or Solanum of the family Solanaceae, examples of different-family plants grafted therewith preferably include plants belonging to the family Brassicaceae, Capparaceae, Capparidaceae, Asteraceae, Calyceraceae, Goodeniaceae, Menyanthaceae, Stylidiaceae, Apocynaceae, Gentianaceae, Leguminosae, Polygalaceae and Surianaceae, and more preferably include plants belonging to the family Brassicaceae, Asteraceae, Apocynaceae and Leguminosae.

In the case where the delivery medium of the present invention comprises a partial structure of a plant belonging to the family Brassicaceae such as a plant belonging to the genus Arabidopsis, examples of different-family plants grafted therewith preferably include plants belonging to the family Asteraceae, Calyceraceae, Goodeniaceae, Menyanthaceae, Stylidiaceae, Malvaceae, Muntingiaceae, Cytinaceae, Dipterocarpaceae, Sarcolaenaceae, Cistaceae, Bixaceae, Sphaerosepalaceae, Cucurbitaceae, Tetramelaceae, Begoniaceae, Datiscaceae, Ranunculaceae, Berberidaceae, Menispermaceae, Linderniaceae, Lythraceae, Astilbe, Plantaginaceae, Scrophulariaceae, Lamiaceae, Mazaceae, Phrymaceae, Paulowniaceae, Solanaceae, Convolvulaceae, Leguminosae, Polygalaceae, Surianaceae, Apocynaceae, Gentianaceae and Orobanchaceae, and more preferably include plants belonging to the family Asteraceae, Malvaceae, Cucurbitaceae, Ranunculaceae, Linderniaceae, Plantaginaceae, Lamiaceae, Solanaceae, Convolvulaceae, Leguminosae, Apocynaceae and Orobanchaceae.

In the case where the delivery medium of the present comprises a partial structure of a plant belonging to the family Lamiaceae such as plants belonging to the genus Perilla, examples of different-family plants grafted therewith preferably include plants belonging to the family Brassicaceae, Capparaceae, Capparidaceae, Leguminosae, Polygalaceae, Surianaceae, Asteraceae, Calyceraceae, Goodeniaceae, Menyanthaceae, Stylidiaceae, Cucurbitaceae, Tetramelaceae, Begoniaceae, Datiscaceae, Solanaceae, Convolvulaceae, Saururaceae, Piperaceae, Apocynaceae, Gentianaceae, Buxaceae, Didymellaceae, Orobanchaceae, Mazaceae, Phrymaceae and Paulowniaceae, and more preferably include plants belonging to the family Brassicaceae, Leguminosae, Asteraceae, Cucurbitaceae, Solanaceae, Saururaceae, Apocynaceae, Buxaceae and Orobanchaceae.

In the case where the delivery medium of the present invention comprises a partial structure of a plant belonging to the family Orobanchaceae, such as a plant belonging to the genus Phtheirospermum, examples of different-family plants grafted therewith include plants belonging to the family Apocynaceae, Gentianaceae, Asteraceae, Calyceraceae, Goodeniaceae, Menyanthaceae, Stylidiaceae, Leguminosae, Polygalaceae, Surianaceae, Buxaceae, Didymellaceae, Saururaceae, Piperaceae, Dryopteridaceae, Hypodematiaceae, Lomariopsidaceae, Nephrolepidaceae, Tectariaceae, Oleandraceae, Polypodiaceae, Davalliaceae, Cucurbitaceae, Tetramelaceae, Begoniaceae, Datiscaceae, Solanaceae, Convolvulaceae, Brassicaceae, Capparaceae and Capparidaceae, and more preferably include plants belonging to the family Apocynaceae, Asteraceae, Leguminosae, Buxaceae, Saururaceae, Dryopteridaceae, Cucurbitaceae, Solanaceae, and Brassicaceae.

There are no particular limitations on the virus for producing a characteristic regulator that is delivered to a different-family plant through the delivery vehicle of the present invention provided it is a plant virus capable of producing a characteristic regulator in a plant body.

There are no particular limitations on the characteristic regulator provided it is able to be produced from a plant virus. Examples of characteristic regulators include RNA and protein. RNA also includes non-coding RNA such as siRNA. Examples of siRNA target genes, RNA-derived genes and protein-derived genes include genes belonging to the PEBP gene family (such as FT gene or TFL1 gene), genes belonging to the MADS gene family (such as SOC1 gene, AP1 gene, AP3 gene, AG gene or FLC gene), genes belonging to the LFY gene family (such as LFY gene or FLO gene), genes belonging to the bZIP gene family (such as FD gene or ABI5 gene), genes belonging to the bHLH gene family (such as PIF3 gene or SPCH gene), genes belonging to the AP2 gene family (such as AP2 gene or PLT gene), genes belonging to the ARF gene family (such as ETT gene or MP gene), genes belonging to the BZR gene family (such as BZR1 gene or BES1 gene), genes belonging to the CCT gene family (such as CO gene or TOC1 gene), genes belonging to the Dof gene family (such as CDF1 gene or OBP1 gene), genes belonging to the YABBY gene family (such as FIL gene or CRC gene), genes belonging to the C2H2-zinc finger gene family (such as JAG gene, KNU gene or SUP gene), genes belonging to the G2-like gene family (such as KAN1 gene, LUX gene or APL gene), genes belonging to the GRAS gene family (such as RGA1 gene or LAS gene), genes belonging to the GRF gene family, genes belonging to the Homeobox gene family (such as BEL1 gene belonging to the BEL gene subfamily, LMI1 gene belonging to the HD-zip subfamily, HAT1 gene, PHB gene, ATML1 gene, STM gene, BP gene or KNAT3 gene belonging to the KNOX subfamily, or WUS gene or WOX9 gene belonging to the WOX subfamily), genes belonging to the HSF gene family, genes belonging to the MYB gene family (such as GL1 gene or RAX1 gene), genes belonging to the MYB-related gene family (such as CCA1 gene or CPC gene), genes belonging to the NAC gene family (such as CUC1 gene or VND6 gene), genes belonging to the PHD gene family (such as MS1 gene), genes belonging to the SBP gene family (such as SPL3 gene or SPL9 gene), genes belonging to the TCP gene family (such as Tb1 gene, CYC gene or CIN gene), genes belonging to the Trihelix gene family (such as PTL gene), VOZ gene, genes belonging to the WRKY gene family, TAW1 gene, genes belonging to the BTB/POZ gene family (such as BOP1 gene), genes encoding peptide hormones (such as CLE, RGF/CLEL/GLV, PSK, PSY, CEP, IDA, SCRL, LURE, EPF, RALF or ESF1), genes belonging to the LRR-RK gene family (such as CLV1 gene or ER gene), genes belonging to the F-box gene family (such as UFO gene or FKF gene), biosynthesis genes of plant hormones (such as auxin, cytokinin, gibberellin, ethylene, abscisic acid, brassinosteroid, jasmonic acid, salicylic acid or strigolactone) (such as YUC gene, IPT gene, GAox gene, ACS gene, NCED gene, DWF4 gene, DAD1 gene, ICS gene or CCD gene), genes encoding receptors (such as TIR1 gene, AHK2 gene, GID1 gene, ETR1 gene, PYR gene, BRI1 gene, COI1 gene, NPR3 gene or D14 gene), genes involved in signal transduction (such as Aux/IAA gene, ARR gene, DELLA gene, CTR1 gene, PP2A gene, BSK1 gene, JAZ gene, NPR1 gene or SMAX1 gene), genes encoding photoreceptors (such as PHY gene, CRY gene or PHOT gene), genes encoding micro RNA (such as MIR156 gene or MIR165 gene), and genes encoding enzymes involved in biosynthesis of primary or secondary metabolites (such as FAD2 gene or GAD gene).

Furthermore, in the present description, "production" refers to, for example, in the case where the characteristic regulator is siRNA, the formation of siRNA resulting from the cleavage of the genome of a plant virus for producing a characteristic regulator in a plant, or the formation of siRNA caused by the transcription of RNA from the genome of a plant virus for producing a characteristic regulator, and cleavage of dsRNA (double-stranded RNA) formed from that RNA; and in the case where the characteristic regulator is a protein, refers to the formation of a protein resulting from the transcription of mRNA from the genome of a plant virus for producing a characteristic regulator, and subsequent translation of that mRNA.

Furthermore, in the present description, the term "characteristic" refers to not only characteristics inherited by the next generation of a certain plant, but also includes characteristics expressed only in the current generation of a certain plant.

There are no particular limitations on the plant virus provided it is able to infect the delivery medium of the present invention and a plant grafted therewith as well as express a characteristic regulator in a different-family plant to which the plant virus is delivered through the delivery medium of the present invention. Examples of plant viruses include cucumber mosaic virus, African casaba mosaic virus, apple latent spherical virus, barley stripe mosaic virus, bean pod mottle virus, beet curly top virus, brome mosaic virus, cabbage leaf curl virus, cotton leaf crumple virus, cymbidium mosaic virus, grapevine virus A, pea early browning virus, poplar mosaic virus, potato virus X, rice tungro bacilliform virus, satellite tobacco mosaic virus, tobacco curly shoot virus, tobacco mosaic virus, tobacco rattle virus, tomato mosaic virus, foxtail mosaic virus, pepino mosaic virus, narcissus mosaic virus, bean yellow dwarf virus, tobacco yellow dwarf virus, wheat dwarf virus, maize streak virus, barley stripe mosaic virus, cowpea mosaic virus, beet yellows virus, beet western yellows virus and white clover mosaic virus. Preferable combinations of plant viruses and plants are reported in various literature, and are reported in, for example, Reference a (Trends in Plant Science, December 2011, Vol. 16, No. 12, pp. 656-665) and Reference b (Virus, Vol. 60, No. 2, pp. 156-162, 2010). The plant virus can be suitably selected based on the information contained in these references.

The genome of the plant virus for producing a characteristic regulator contains a region for expressing a characteristic regulator in addition to the genomic region of the plant virus. Examples of this region in the case where the characteristic regulator is siRNA include a promoter sequence of a target gene of the siRNA, a sequence encoding a target gene of the siRNA, and a sequence required to express the siRNA (such as a promotor sequence that functions in plants or a sequence that encodes RNA that forms the siRNA as a result of being cleaved). Other examples of this region in the case where the characteristic regulator is a protein include a sequence required to express the protein (such as a promoter sequence that functions in plants or a sequence that encodes the protein). Other examples of this region include sequences for expressing a characteristic regulator as a fusion gene with a viral gene. In the case where the characteristic regulator is siRNA, the characteristic regulator can be expressed in a form in which the transcription product of a viral gene is fused with, for example, a promoter sequence of a target gene of the siRNA, a sequence that encodes a target gene of the siRNA, or a sequence that encodes RNA that forms the siRNA as a result of being cleaved. In the case where the characteristic regulator is a protein, although the characteristic regulator can also be expressed as a fusion protein of the protein and a viral protein, the protein can be expressed in a state of being separated from the viral protein, by inserting a peptidase recognition sequence (such as a 2A peptide sequence) into a linker between the protein and viral protein. In addition, the protein can also be expressed as an independent protein by a subgenomic promoter on the virus genome.

Although the delivery medium of the present invention is not required to contain a plant virus for producing a characteristic regulator only if it is used to regulate a characteristic by delivering the plant virus for producing a characteristic regulator to a different-family plant through the delivery medium, as is explained in the section entitled, "3. Characteristic-Regulating Method", to be subsequently described, from the viewpoint of being able to deliver a virus easily, the delivery medium of the present invention preferably comprises a plant virus for producing a characteristic regulator. Although there are no particular limitations on the amount of virus contained, specific examples thereof include 0.05 fg to 100 mg, preferably 50 fg to 100 mg, more preferably 50 pg to 100 mg and even more preferably 50 pg to 10 mg per one delivery medium of the present invention.

The delivery medium of the present invention can be produced in accordance or in compliance with known methods.

As a first example of the method for producing the delivery medium of the present invention (Production Example 1), in the case where the delivery medium of the present invention comprises only one type of partial structure of an inter-family-graftable plant, the inter-family-graftable plant is cultivated in accordance with established methods, followed by cutting out a portion thereof.

As a second example of the method for producing the delivery medium of the present invention (Production Example 2), in the case where the delivery medium of the present invention comprises two types of partial structures of inter-family-graftable plants, after having produced a plant body obtained by grafting the two types of inter-family-graftable plants, sites containing each of the two types of partial structures are cut out.

As a third example of the method for producing the delivery medium of the present invention (Production Example 3), in the case where the delivery medium of the present invention comprises a plant virus for producing a characteristic regulator, after having infected an inter-family-graftable plant or a plant containing a partial structure thereof with the plant virus for producing a characteristic regulator, the portion containing the partial structure of the inter-family-graftable plant is cut out.

Furthermore, in the present invention, infection with the plant virus for producing a characteristic regulator can be carried out in accordance or in compliance with known methods.

A first example of the method for infecting with a plant virus for producing a characteristic regulator (Infection Example 1) is the following method: a method comprising preparing a plasmid containing a promoter (such as a T7 promoter, T3 promoter or 35S promoter) and the genome of a plant virus for producing a characteristic regulator located downstream therefrom (containing the genomic region of the plant virus and the region for expressing a characteristic regulator) (Step a1); obtaining genomic RNA of the plant virus for producing a characteristic regulator from the plasmid obtained in Step a1 by in vitro transcription (Step b1); and inoculating a plant with the genomic RNA obtained in Step b1 (by, for example, friction inoculation or particle gun inoculation)(Step c1). Alternatively, in the case where the plasmid obtained in Step a1 is a Ti plasmid containing a promoter that has the ability to activate transcription within plant cells, such as a 35S promoter, infection can be carried out by a method comprising introducing the plasmid obtained in Step a1 into Agrobacterium followed by culturing (Step b2), and inoculating a plant with the culture solution obtained in Step b2 (by, for example, infiltration, toothpick inoculation or aspiration injection) (Step c2), instead of the aforementioned Steps b1 and c1. Alternatively, infection can be carried out by a method comprising inoculating a plant with the plasmid obtained in Step a1 (by, for example, friction inoculation or particle gun inoculation) (Step c3), instead of the above-mentioned Steps b1 and c1. Alternatively, infection can be carried out by a method comprising creating a stable transformant (by, for example, the leaf disk method, influorescence infiltration method or vacuum filtration method) (Step c4), instead of the above-mentioned Step c2. A plant virus for producing a characteristic regulator can be produced from genomic RNA, a plasmid or T-DNA introduced into a plant according to these methods.

A second example of the method for infecting with a plant virus for producing a characteristic regulator (Infection Example 2) is the following method: a method comprising collecting a plant virus for producing a characteristic regulator from a plant containing that virus (such as that obtained according to the above-mentioned Infection Example 1) (Step d1), and inoculating another plant with the virus collected in Step d1 (Step e1). The collection of the virus in Step d1 can be carried out by, for example, recovering a virus liquid obtained by pulverizing a portion of a plant (such as a leaf) containing the plant virus for producing a characteristic regulator. The inoculation of Step e1 can be carried out by making a scratch at the site of the plant targeted for inoculation (such as a leaf) using an abrasive such as silicon carbide, and bringing the virus into contact with the site.

### 2. Plant Body

In one aspect thereof, the present invention relates to a partial structure obtained by grafting the delivery medium of the present invention with a different-family plant either directly or through another plant (which may also be indicated as the "partial structure of the present invention" in the present description), and a plant body containing the partial structure of the present invention (which may also be indicated as the "plant body of the present invention" in the present description). The following provides an explanation thereof.

The different-family plant in the partial structure of the present invention is as defined for the "different-family plant grafted with the delivery medium of the present invention" explained in the "1. Delivery Medium" section above.

The partial structure of the present invention includes a partial structure obtained by grafting the delivery medium of the present invention with a different-family plant directly, and a partial structure obtained by grafting the delivery medium of the present invention with a different-family plant through another plant. In the latter case, there are no particular limitations on "another plant," and it may be a plant of the same family as the partial structure of an inter-family-graftable plant contained in the delivery medium of the present invention. Specific examples of the partial structure of the present invention include a partial structure obtained by grafting the delivery medium of the present invention and a different-family plant in that order starting from one side of a plant (such as the tip of any root, stalk, branch or leaf), and a partial structure obtained by grafting the delivery medium of the present invention, another plant and a different-family plant in that order starting from one side of the plant (such as the tip of any root, stalk, branch or leaf).

The plant body of the present invention includes a plant body composed only of the partial structure of the present invention, and a plant body obtained by grafting the partial structure of the present invention with one or a plurality of other plants. In the latter case, there are no particular limitations on the "other plants." Specific examples of the plant body of the present invention include a plant body obtained by grafting the delivery medium of the present invention and a different-family plant in that order starting from one side of the plant (such as the tip of any root, stalk, branch or leaf), a plant body obtained by grafting the partial structure of the present invention and one or a plurality of other plants in that order starting from onside of the plant (such as the tip of any root, stalk, branch or leaf), and a plant body obtained by grafting one or a plurality of other plants, the partial structure of the present invention and one or a plurality of other plants in that order starting from one side of the plant (such as the tip of any root, stalk, branch or leaf).

### 3. Characteristic-Regulating Method

In one aspect thereof, the present invention relates to a plant body characteristic-regulating method comprising delivering a plant virus for producing a characteristic regulator to the plant body of the present invention through the delivery medium of the present invention contained in the plant body (which may also be indicated as the "characteristic-regulating method of the present invention" in the present description). In addition, the present invention also relates to a method for producing a characteristic-regulated plant body comprising this step. The following provides an explanation thereof.

According to the characteristic-regulating method of the present invention, a plant virus for producing a characteristic regulator can be delivered to a different-family plant through the delivery medium of the present invention, enabling the characteristic regulator to be expressed in the different-family plant.

There are no particular limitations on the method for delivering the plant virus for producing a characteristic regulator provided the virus is delivered through the delivery medium of the present invention. In addition to the method for infecting the site of the delivery medium of the present invention in the plant body of the present invention with a plant virus for producing a characteristic regulator, in the case where the plant body of the present invention has a structure obtained by grafting two plants (with at least one being a different-family plant) either directly or indirectly through the delivery medium of the present invention, the delivery method includes a method for infecting a site other than the delivery medium of the present invention with a plant virus for producing a characteristic regulator. Even in the latter case, if one side is infected with the plant virus for producing a characteristic regulator through the delivery medium of the present invention, the virus is also delivered to the other side (the side of the different-family plant) through the delivery medium of the present invention.

As has been described above, in addition to infecting the plant body of the present invention with a virus, in the case where the delivery medium of the present invention contains a plant virus for producing a characteristic regulator, as a result of grafting the delivery medium of the present invention with a different-family plant either directly or through another plant, the plant virus for producing a characteristic regulator can be delivered to the resulting plant body of the present invention through the delivery medium of the present invention. This method makes it possible to deliver a virus more easily. In this method, once grafting is made, the virus in the delivery medium of the present invention is delivered to a different-family plant and a characteristic regulator can be expressed in the different-family plant.

### 4. Seed of Characteristic-Regulated Plant Body

In one aspect thereof, the present invention relates to a seed of a characteristic-regulated plant body obtained from the plant body of the present invention or an offspring plant body of the plant body of the present invention (which may also be indicated as the "seed of the present invention" in the present description).

The seed of the present invention is a seed obtained from the plant body of the present invention for which a characteristic has been regulated according to the characteristic-regulating method of the present invention, or from an offspring plant body of the plant body; as long as the seed is so, there are no particular limitations thereon. Here, the offspring plant body includes not only an F1 plant body obtained by germination of a seed of a certain F0 plant body, but also subsequent generations of the F1 plant body (subsequent generations, like F2, F3, F3 etc.).

### 5. Seed Group of Characteristic-Regulated Plant Body

In one aspect thereof, the present invention relates to a seed group of a characteristic-regulated plant body obtained from the plant body of the present invention or an offspring plant body of the plant body of the present invention (which may be also be indicated as the "seed group of the present invention" in the present description). The following provides an explanation thereof.

The seed group of the present invention is a seed group obtained from the plant body of the present invention for which a characteristic has been regulated according to the characteristic-regulating method of the present invention, or from an offspring plant body of the plant body; as long as the seed group is so, there are no particular limitations thereon. The offspring plant body is as defined in the above-mentioned section entitled, "4. Seed of Characteristic-Regulated Plant Body".

A characteristic of the seed group of the present invention is efficiently regulated as a result of being obtained from the plant body of the present invention for which a characteristic has been regulated according to the characteristic-regulating method of the present invention, or from an offspring plant body of the plant body. Namely, in the seed group of the present invention, there is less damage to genomic DNA than in those obtained by conventional characteristic-regulating methods requiring individual regeneration.

### Examples

Although the following provides a detailed explanation of the present invention based on examples thereof, the present invention is not limited by these examples.

### Example 1: Virus Delivery Test 1

Virus-infected Nicotiana benthamiana and Arabidopsis thaliana, belonging to a different family therefrom, were grafted followed by investigating whether or not the plant virus is detected in tissue of Arabidopsis thaliana after the passage of a certain amount of time. Detection of the plant virus indicates that the virus migrated to the different-family plant through Nicotiana benthamiana. More specifically, this was carried out in the manner described below.

### <Example 1-1: Preparation of Virus-Infected Nicotiana benthamiana>

Nicotiana benthamiana seeds were planted in culture soil followed by cultivating for one to two months on a plant cultivation shelf under conditions of continuous constant lighting. The leaves of a separately prepared plant previously infected with cucumber mosaic virus (CMV) were pulverized to a liquid in 0.1 M phosphate buffer containing an oxidase inhibitor in the form of 10 mM DIECA (diethyldithiocarbamate) to prepare a virus liquid. Following cultivation, carborundum (silicon carbide) was sprinkled onto an arbitrary leaf of Nicotiana benthamiana followed by dropping 6 µl of the virus liquid thereon and rubbing the entire surface of the leaf. The surface of the inoculated leaf was rinsed with tap water followed by cultivating on the plant cultivation shelf for an additional week under conditions of continuous constant lighting. The resulting plant body was subjected to the grafting treatment indicated below.

### <Example 1-2: Preparation of Arabidopsis thaliana>

Arabidopsis thaliana seeds were planted in culture soil followed by cultivating for several weeks to several months on a plant cultivation shelf under conditions of continuous constant lighting. The resulting plant body was subjected to the grafting treatment indicated below.

### <Example 1-3: Grafting Treatment and Sampling>

Grafting treatment was carried out using the virus-infected Nicotiana benthamiana as budwood and Arabidopsis thaliana as rootstock. More specifically, grafting treatment was carried out in the manner indicated below. Grafting treatment (cleft grafting) was carried out on the stalk. The rootstock was prepared by severing the stalk horizontally, and making a notch measuring about 1 cm to 2 cm in the center of the cross-sectional surface. The budwood was prepared by severing the stalk and cutting away the upper portion followed by making a V-shape at the cut site so as to fit into the notch of the rootstock. This series of cuts was made using a single-edged razor. The stalk of the budwood was gently inserted into the notch made in the stalk or petiole of the rootstock so as not to damage the stalk of the budwood, and the budwood was immobilized with Parafilm to prevent it from shifting out of position. Support poles were attached to the rootstock and budwood, a plastic bag sprayed with water was placed over the budwood so as to cover the entire budwood, and finally the zipper of the plastic bag was closed to the side where the stalk of the rootstock was located. The grafted plant was grown for 7 days while in this state in an incubator under conditions of 27°C, low light and continuous constant lighting, and on day 7, an incision was made in the plastic bag and the lower zipper was also opened followed by allowing to stand for one additional day. On the following day, the plastic bag was removed after confirming that the water inside had evaporated. Subsequently, growing was continued on a cultivation shelf under conditions of 24°C and continuous constant lighting, and new tissue away from the grafting site of the non-CMV-infected individual was sampled on days 8 to 20 after grafting. In addition, sampling was also carried out in the same manner in cases in which grafts were not established due to technical problems with the grafting treatment.

### <Example 1-4: Virus Detection>

Virus was detected by RT-PCR using a primer specific for the nucleobase sequence of the virus. More specifically, virus detection was carried out in the manner indicated below. In addition to the sample obtained in the above-mentioned Example 1-3, a sample of virus-infected Nicotiana benthamiana was used as a positive control and non-virus-infected Arabidopsis thaliana was used as a negative control. Total RNA was extracted from each sample using a TRIzol® Reagent (Life Technologies), cDNA was synthesized from the RNA using SuperScriptIII (Life Technologies), and PCR reactions were carried out for 30 to 32 cycles using this as a template. Primer sets used in the PCR reaction were as follows: a primer set for detecting CMV (CPTALL-3: GACTGACCATTTTAGCCG (SEQ ID NO: 1) and CPTALL-5: YASYTTTDRGGTTCDAATTCC (SEQ ID NO: 2)), a primer set specific for Arabidopsis thaliana actin gene (internal standard) (AtACT2_F: TTCAGATGCCCAGAAGTCTTGTTCC (SEQ ID NO: 3) and AtACT2_R: CTGGACCTGCCTCATCATACTC (SEQ ID NO: 4)), and a primer set specific for Nicotiana benthamiana actin gene (internal standard) (NbACT_F: CCTGAGGTCCTTTTCCAACCATC (SEQ ID NO: 5) and NbACT_R: GCCTTTGCAATCCACATCTGTTGG (SEQ ID NO: 6)). The PCR products were electrophoresed using agarose gel and the gel was stained with ethidium bromide to confirm the band patterns.

### <Example 1-5: Results>

Stained images of the electrophoresed gel obtained in Example 1-4 are shown in Fig. 1. As shown in Fig. 1, a band specific for CMV was detected in the Arabidopsis thaliana grafted with the virus-infected Nicotiana benthamiana (Nb(+)/At(-)) as in the positive control (Nb(+)). On the other hand, a CMV-specific band was not detected in the negative control (At(-)). This indicates that a virus can be delivered to a plant (rootstock) of a different family through Nicotiana benthamiana (budwood).

### Example 2: Virus Delivery Test 2

Whether or not a virus migrates to a different-family plant from virus-infected Nicotiana benthamiana was investigated using a virus expression vector and n Agrobacterium. More specifically, this was carried out in the manner indicated below.

### <Example 2-1: Preparation of Virus-Infected Nicotiana benthamiana>

Nicotiana benthamiana seeds were planted in culture soil followed by cultivating for one to two months on a plant cultivation shelf under conditions of continuous constant lighting. The vector used for infection was produced by artificially synthesizing the genome sequence of apple latent spherical virus (ALSV). The RNA1 or RNA2 sequence of ALSV was introduced between the cauliflower mosaic virus (CaMV) 35S promoter and nopaline synthase terminator of pGWB2 vector to obtain pALSV1 or pALSV2, respectively. At this time, the 5'-terminal of RNA1 or RNA2 of ALSV was aligned with the transcription starting point of the CaMV35S promoter. Infection with ALSV was carried out using an Agrobacterium (GV3101 (strain pMP90)). pALSV1 or pALSV2 was introduced into the Agrobacterium by electroporation. After screening with an LB medium containing necessary antibiotics, the Agrobacterium was pre-cultured overnight at 28°C in 2 ml of an LB medium containing necessary antibiotics. 0.5 ml of the pre-culture solution was transferred to 10 ml of an LB medium containing necessary antibiotics followed by culturing for 5 to 6 hours at 28°C until an OD600 of 0.5 to 0.6 was achieved. The cells were harvested by centrifugation and re-suspended in 10 ml of an Agrobacterium induction buffer (composition: 10 mM MES (pH 5.5), 100 µM acetosyringone). The suspension was shaken for 3 hours at room temperature and 50 rpm. Subsequently, the cells were harvested by centrifugation and re-suspended in an infiltration buffer (composition: 5 mM MES (pH 5.5)). The Agrobacterium incorporating pALSV1 and the Agrobacterium incorporating pALSV2 were mixed to give an equal concentration. The mixed infection solution was injected into leaves of Nicotiana benthamiana using a syringe. The plant was cultivated for an additional one week on a plant cultivation shelf under conditions of continuous constant lighting. The resulting plant body was subjected to the grafting treatment indicated below.

### <Example 2-2: Preparation of Arabidopsis thaliana>

Arabidopsis thaliana seeds were planted in culture soil followed by cultivating for several weeks to several months on a plant cultivation shelf under conditions of continuous constant lighting. The resulting plant body was subjected to the grafting treatment indicated below.

### <Example 2-3: Grafting Treatment and Sampling>

Grafting treatment was carried out using a virus-infected Nicotiana benthamiana as budwood and Arabidopsis thaliana as rootstock. More specifically, grafting treatment was carried out in the manner indicated below. Grafting treatment (cleft grafting) was carried out on the stalk. The rootstock was prepared by severing the stalk horizontally, and making a notch measuring about 1 cm to 2 cm in the center of the cross-sectional surface. The budwood was prepared by severing the stalk and cutting away the upper portion followed by cutting it into a V-shaped at the cut site so as to fit into the notch of the rootstock. This series of cuts was made using a single-edged razor. The stalk of the budwood was gently inserted into the notch made in the stalk or petiole of the rootstock so as not to damage the stalk of the budwood, and the budwood was immobilized with Parafilm to prevent it from shifting out of position. Support poles were attached to the rootstock and budwood, a plastic bag sprayed with water was placed over the budwood so as to cover the entire budwood, and finally the zipper of the plastic bag was closed to the side where the stalk of the rootstock was located. The grafted plant was grown for 7 days while in this state in an incubator under conditions of 27°C, low light and continuous constant lighting, and on day 7, an incision was made in the plastic bag and the lower zipper was also opened followed by allowing to stand for one additional day. On the following day, the plastic bag was removed after confirming that the water inside had evaporated. Subsequently, growing was continued on a cultivation shelf under conditions of 24°C and continuous constant lighting, and new tissue away from the grafting site of the non-ALSV-infected individual was sampled on days 8 to 20 after grafting.

### <Example 2-4: Virus Detection>

Virus was detected by RT-PCR using a primer specific for the nuclebase sequence of the virus. More specifically, virus detection was carried out in the manner indicated below. In addition to the sample obtained in the above-mentioned Example 2-3, Arabidopsis thaliana grafted with non-virus-infected Nicotiana benthamiana was used as a negative control. Total RNA was extracted from each sample using a TRIzol® Reagent (Life Technologies), cDNA was synthesized from the RNA using SuperScriptIII (Life Technologies), and PCR reactions were carried out using this as a template, 32 cycles for ALSV detection and 28 cycles for the internal standard. Primer sets used in the PCR reaction are as follows: a primer set for detecting ALSV (ALSV_F: GAGTGGTCATGCTACCATTCGAG (SEQ ID NO: 7) and ALSV_R: CAGGTGAACTTTCCCCAAAAGCTTG (SEQ ID NO: 8)) and a primer set specific for Arabidopsis thaliana actin gene (internal standard) (AtACT2_F: TTCAGATGCCCAGAAGTCTTGTTCC (SEQ ID NO: 3) and AtACT2_R: CTGGACCTGCCTCATCATACTC (SEQ ID NO: 4)). The PCR products were electrophoresed using agarose gel and the gel was stained with ethidium bromide to confirm the band patterns.

### <Example 2-5: Results>

Stained images of the electrophoresed gel obtained in Example 2-4 are shown in Fig. 2. As shown in Fig. 2, a band specific for ALSV was detected in Arabidopsis thaliana grafted with virus-infected Nicotiana benthamiana (Nb(+)/At(-)). On the other hand, this band was not detected in the negative control (Nb(-)/At(-)). This indicates that a virus can be delivered to a plant (rootstock) of a different family through Nicotiana benthamiana (budwood) even when a virus expression vector was used to infect Nicotiana benthamiana with the virus.

### Example 3: Virus Delivery Test 3

Virus-infected Nicotiana benthamiana and Brassica napus, belonging to a different family therefrom, were grafted followed by investigating whether or not the plant virus is detected in tissue of Brassica napus after the passage of a certain amount of time. Detection of the plant virus indicates that the virus has migrated to the different-family plant through Nicotiana benthamiana. More specifically, this was carried out in the manner described below.

### <Example 3-1: Preparation of Virus-Infected Nicotiana benthamiana>

Nicotiana benthamiana seeds were planted in culture soil followed by cultivating for one to two months on a plant cultivation shelf under conditions of continuous constant lighting. Infection with tobacco rattle virus (TRV) was carried out using an Agrobacterium (GV3101 (strain pMP90)). pTRV1 and pTRV2-MCS vectors were used to infect with TRV. The Agrobacterium was prepared in the same manner as in Example 2-1, and the Agrobacterium incorporating pTRV1 and the Agrobacterium incorporating pTRV2-MCS were mixed to give an equal concentration. The mixed infection solution was injected into leaves of Nicotiana benthamiana using a syringe. The plant was cultivated for an additional one week on a plant cultivation shelf under conditions of continuous constant lighting. The resulting plant body was subjected to the grafting treatment indicated below.

### <Example 3-2: Preparation of Brassica napus>

Brassica napus seeds were planted in culture soil followed by cultivating for several weeks on a plant cultivation shelf under conditions of continuous constant lighting at 22°C. Vernalization was carried out for 6 weeks or more at 4°C to promote elongation of flower stems. Subsequently, cultivation was again carried out on a plant cultivation shelf under conditions of continuous constant lighting at 22°C for several weeks. The resulting plant body was subjected to the grafting treatment indicated below.

### <Example 3-3: Grafting Treatment and Sampling>

Grafting treatment was carried out in the same manner as in Example 1-3 using the virus-infected Nicotiana benthamiana as budwood and Brassica napus as rootstock. Following grafting treatment, the grafted plant was grown for 7 days in an incubator under conditions of 27°C, low light and continuous constant lighting, and on day 7, an incision was made in the plastic bag and the lower zipper was also opened followed by allowing to stand for one additional day. On the following day, the plastic bag was removed after confirming that the water inside had evaporated. Subsequently, the grafted plant was treated for 7 days at 4°C to promote viral infection. Growing was again continued on a cultivation shelf under conditions of 22°C and continuous constant lighting, and new tissue away from the grafting site of the non-TRV-infected individual was sampled on days 14 to 20 after grafting.

### <Example 3-4: Virus Detection>

Virus was detected by RT-PCR using a primer specific for the nucleobase sequence of the virus. More specifically, virus detection was carried out in the manner indicated below. In addition to the sample obtained in the above-mentioned Example 3-3, a sample of virus-infected Nicotiana benthamiana was used as a positive control and non-virus-infected Brassica napus was used as a negative control. Total RNA was extracted from each sample using a TRIzol® Reagent (Life Technologies), cDNA was synthesized from the RNA using SuperScriptIII (Life Technologies), and PCR reactions were carried out using this as a template, 32 cycles for TRV detection and 28 cycles for the internal standard. Primer sets used in the PCR reaction are as folllos: a primer set 1 for detecting TRV (TRV2_F: CTGGGAGATGATACGCTGTTTGAG (SEQ ID NO: 9) and TRV2_R: GAACCTAAAACTTCAGACACGGATCTAC (SEQ ID NO: 10)), a primer set specific for Brassica napus actin gene (internal standard) (BnaACT2_F: CTGTCCCAATCTACGAGGGTTTC (SEQ ID NO: 11) and BnaATC2_R: GCTCTGCTGTCGTGGTGAAC (SEQ ID NO: 12)), and a primer set specific for Nicotiana benthamiana actin gene (internal standard) (NbACT_F: CCTGAGGTCCTTTTCCAACCATC (SEQ ID NO: 5) and NbACT_R: GCCTTTGCAATCCACATCTGTTGG (SEQ ID NO: 6)). The PCR products were electrophoresed using agarose gel and the gel was stained with ethidium bromide to confirm the band patterns.

### <Example 3-5: Results>

A band specific for TRV was detected in the Brassica napus (Nb(+)/Bn(-)) grafted with the virus-infected Nicotiana benthamiana, as detected in the positive control (Nn(+)). On the other hand, a band specific for TRV was not detected in the negative control (Bn(-)). This indicates that a virus can be delivered to a plant (rootstock) of a different family through Nicotiana benthamiana (budwood).

### Example 4: Test of Delivery of siRNA by Virus

Nicotiana benthamiana, infected with a virus containing a partial sequence of an endogenous gene of Arabidopsis thaliana, and Arabidopsis thaliana, belonging to a different family therefrom, were grafted followed by observing the phenotype of the Arabidopsis thaliana following the passage of a fixed amount of time while also investigating the expression level of the target endogenous gene. A change in phenotype accompanying a decrease in expression level of the target endogenous gene indicates that a characteristic of the different-family plant has been regulated through Nicotiana benthamiana. More specifically, this was carried out in the manner described below.

### <Example 4-1: Preparation of Virus-Infected Nicotiana benthamiana>

Nicotiana benthamiana seeds were planted in culture soil followed by cultivating for one to two months on a plant cultivation shelf under conditions of continuous constant lighting. A vector was produced that incorporated a partial sequence of the PDS gene of Arabidopsis thaliana (CATGGTTCCAAGATGGCATTCTTGGATGGTAATCCTCCGGAAAGGCTTTGTATGCCAGTAG TGGATCATATTCGATCACTAGGTGGGGAAGTGCAACTTAATTCTAGGATAAAGAAAATTGAG CTCAATGACGATGGCACGGTTAAGAGTTTCTTACTCACTAATGGAAGCACTGTCGAAGGAGA CGCTTATGTGTTTGCC (SEQ ID NO: 13)) between the MP gene and VP25 gene of RNA2 of ALSV. The PDS gene sequence was inserted so as to match the virus translation frame, and codons of the amino acid sequence extending from glutamine to glycine that recognize cysteine protease (C-PRO) were introduced to both sides. The Agrobacterium was prepared in the same manner as in Example 2-1, and the Agrobacterium incorporating a vector expressing RNA1 of ALSV and the Agrobacterium incorporating a vector expressing RNA2 containing the PDS gene were mixed to give an equal concentration. The mixed infection solution was injected into leaves of Nicotiana benthamiana using a syringe. The plant was cultivated for one week on a plant cultivation shelf under conditions of continuous lighting. The resulting plant body was subjected to the grafting treatment indicated below.

### <Example 4-2: Preparation of Arabidopsis thaliana>

Arabidopsis thaliana seeds were planted in culture soil followed by cultivating for several weeks to several months on a plant cultivation shelf under conditions of continuous lighting. The resulting plant body was subjected to the grafting treatment indicated below.

### <Example 4-3: Grafting Treatment, Observation of Phenotype and Sampling>

Grafting treatment was carried out in the same manner as in Example 1-3 using the virus-infected Nicotiana benthamiana as budwood and Arabidopsis thaliana as rootstock. Following grafting treatment, the grafted plant was grown for 7 days in an incubator under conditions of 27°C, low light and continuous constant lighting, and on day 7, an incision was made in the plastic bag and the lower zipper was also opened followed by allowing to stand for one additional day. On the following day, the plastic bag was removed after confirming that the water inside had evaporated. Subsequently, growing was continued on a cultivation shelf under conditions of 24°C and continuous constant lighting followed by observation of the phenotype for two to three weeks after grafting. In addition, tissue in which the bleaching phenotype was observed was sampled.

### <Example 4-4: Analysis of Expression of PDS Gene>

Expression of PDS gene was analyzed by RT-PCR using a primer specific for the nucleobase sequence of the PDS gene. In addition to the sample obtained in the above-mentioned Example 4-3, tissue samples of non-virus-infected Arabidopsis thaliana and Arabidopsis thaliana in which the bleaching phenotype was not observed were used as negative controls. Total RNA was extracted from each sample using a TRIzol® Reagent (Life Technologies), cDNA was synthesized from the RNA using SuperScriptIII (Life Technologies), and PCR reactions were carried out using this as a template, 30 to 32 cycles. Primer sets used in the PCR reaction are as follows: a primer set 1 specific for PDS gene (AtPDS_F: GATAGCTTTGAACCGGTTTCTTCAGG (SEQ ID NO: 14) and AtPDS_R: GAAGTACGGTATTTCTTTCCAGGGATC (SEQ ID NO: 15)) and a primer set specific for Arabidopsis thaliana actin gene (internal standard) (AtACT2_F: TTCAGATGCCCAGAAGTCTTGTTCC (SEQ ID NO: 3) and AtACT2_R: CTGGACCTGCCTCATCATACTC (SEQ ID NO: 4)). The PCR products were electrophoresed using agarose gel and the gel was stained with ethidium bromide to confirm the band patterns.

### <Example 4-5: Results>

A photograph of the plant bodies obtained in Example 4-3 is shown in Fig. 3. In addition, stained images of electrophoresed gel obtained in Example 4-4 are shown in Fig. 4. As shown in Fig. 3, bleaching of flower stems was observed in the Arabidopsis thaliana grafted with the virus-infected Nicotiana benthamiana. The expression level of PDS gene decreased in the bleached flower stems in comparison with Arabidopsis thaliana that was not subjected to grafting treatment, and tissue in which bleaching did not occur (Fig. 4). This indicates that it is possible to deliver a virus containing siRNA to a plant of a different family (rootstock) through Nicotiana benthamiana (budwood) to regulate a characteristic.

### Example 5: Test of Delivery of Exogenous Protein by Virus

Nicotiana benthamiana, infected with a virus containing the sequence of an exogenous gene in the form of EGFP gene, and Arabidopsis thaliana, belonging to a different family therefrom, were grafted followed by observing EGFP fluorescence in the Arabidopsis thaliana following the passage of a fixed amount of time. The observation of a fluorescence signal indicates that the exogenous protein has migrated to the different-family plant through Nicotiana benthamiana. More specifically, this was carried out in the manner described below.

### <Example 5-1: Preparation of Virus-Infected Nicotiana benthamiana>

Nicotiana benthamiana seeds were planted in culture soil followed by cultivating for one to two months on a plant cultivation shelf under conditions of continuous constant lighting. A vector was produced that incorporated the EGFP gene sequence between the MP gene and VP25 gene of RNA2 of ALSV. The EGFP gene sequence was inserted so as to match the virus translation frame, and codons of the amino acid sequence extending from glutamine to glycine that recognize cysteine protease (C-PRO) were introduced to both sides. The Agrobacterium was prepared in the same manner as in Example 2-1, and the Agrobacterium incorporating a vector expressing RNA1 of ALSV and the Agrobacterium incorporating a vector expressing RNA2 containing the EGFP gene were mixed to give an equal concentration. The mixed infection solution was injected into leaves of Nicotiana benthamiana using a syringe. The plant was cultivated for one week on a plant cultivation shelf under conditions of continuous lighting. The resulting plant body was subjected to the grafting treatment indicated below.

### <Example 5-2: Preparation of Arabidopsis thaliana>

Arabidopsis thaliana seeds were planted in culture soil followed by cultivating for several weeks to several months on a plant cultivation shelf under conditions of continuous lighting. The resulting plant body was subjected to the grafting treatment indicated below.

### <Example 5-3: Grafting Treatment, Observation of Phenotype and Sampling>

Grafting treatment was carried out in the same manner as in Example 1-3 using the virus-infected Nicotiana benthamiana as budwood and Arabidopsis thaliana as rootstock. Following grafting treatment, the grafted plant was grown for 7 days in an incubator under conditions of 27°C, low light and continuous constant lighting, and on day 7, an incision was made in the plastic bag and the lower zipper was also opened followed by allowing to stand for one additional day. On the following day, the plastic bag was removed after confirming that the water inside had evaporated. Subsequently, growing was continued on a cultivation shelf under conditions of 24°C and continuous constant lighting followed by cutting the stalk of the grafted Arabidopsis thaliana two to three weeks after grafting and observing fluorescence.

### <Example 5-4: Results>

Fluorescent micrographs of cross-sections of the plant bodies obtained in Example 5-3 are shown in Fig. 5. As shown in Fig. 5, EGFP fluorescence was observed in phloem tissue of the stalk in the Arabidopsis thaliana grafted with virus-infected Nicotiana benthamiana. This indicates that an exogenous protein can be expressed by delivering a virus containing the exogenous protein to a plant of a different family (rootstock) through Nicotiana benthamiana (budwood).

### Example 6: Test of Delivery of Exogenous Protein by Virus

Nicotiana benthamiana, infected with a virus containing the sequence of an exogenous gene in the form of β-glucuronidase (GUS) gene, and Arabidopsis thaliana, belonging to a different family therefrom, were grafted; and the Arabidopsis thaliana was stained with GUS following the passage of a fixed amount of time. The observation of staining by GUS indicates that the exogenous protein has migrated to the different-family plant through Nicotiana benthamiana. More specifically, this was carried out in the manner described below.

### <Example 6-1: Preparation of Virus-Infected Nicotiana benthamiana>

Nicotiana benthamiana seeds were planted in culture soil followed by cultivating for one to two months on a plant cultivation shelf under conditions of continuous constant lighting. A vector was produced that incorporated the GUS gene sequence between the MP gene and VP25 gene of RNA2 of ALSV. The GUS gene sequence was inserted so as to match the virus translation frame, and codons of the amino acid sequence extending from glutamine to glycine that recognize cysteine protease (C-PRO) were introduced to both sides. The Agrobacterium was prepared in the same manner as in Example 2-1, and the Agrobacterium incorporating a vector expressing RNA1 of ALSV and the Agrobacterium incorporating a vector expressing RNA2 containing the GUS gene were mixed to give an equal concentration. The mixed infection solution was injected into leaves of Nicotiana benthamiana using a syringe. The plant was cultivated for one week on a plant cultivation shelf under conditions of continuous lighting. The resulting plant body was subjected to the grafting treatment indicated below.

### <Example 6-2: Preparation of Arabidopsis thaliana>

Arabidopsis thaliana seeds were planted in culture soil followed by cultivating for several weeks to several months on a plant cultivation shelf under conditions of continuous lighting. The resulting plant body was subjected to the grafting treatment indicated below.

### <Example 6-3: Grafting Treatment, Observation of Phenotype and Sampling>

Grafting treatment was carried out in the same manner as in Example 1-3 using the virus-infected Nicotiana benthamiana as budwood and Arabidopsis thaliana as rootstock. Following grafting treatment, the grafted plant was grown for 7 days in an incubator under conditions of 27°C, low light and continuous constant lighting, and on day 7, an incision was made in the plastic bag and the lower zipper was also opened followed by allowing to stand for one additional day. On the following day, the plastic bag was removed after confirming that the water inside had evaporated. Subsequently, growing was continued on a cultivation shelf under conditions of 24°C and continuous constant lighting followed by staining the grafted Arabidopsis thaliana tissue with GUS two to three weeks after grafting. Arabidopsis thaliana tissue grafted with non-virus-infected Nicotiana benthamiana was also stained in the same manner as a negative control.

### <Example 6-4: GUS Staining>

After subjecting the plant tissue to infiltration treatment for 15 minutes using ice-cold 90% acetone, the plant was immersed in a GUS staining solution (composition: 0.5 mg/mL 5-bromo-4-chloro-3-indolyl-beta delta-glucuronic acid (X-Gluc), 50 mM phosphate buffer (pH 7.2), 5 mM potassium ferrocyanide, 5 mM potassium ferricyanide, 0.2% TritonX-100) followed by repeating a 10- to 15-minute depressurization three times to allow the GUS staining solution to penetrate into the tissue. The sample was stained for 24 hours in a dark place at 37°C. After staining, the reaction was stopped with 70% ethanol.

### <Example 6-5: Results>

Staining with GUSA was observed in the Arabidopsis thaliana grafted with virus-infected Nicotiana benthamiana. Staining was not observed in the Arabidopsis thaliana tissue grafted with non-virus-infected Nicotiana benthamiana serving as a negative control. This indicates that an exogenous protein can be expressed by delivering a virus containing the exogenous protein to a plant of a different family (rootstock) through Nicotiana benthamiana (budwood).

## Claims

1. A characteristic regulation delivery medium for delivering a plant virus for producing a characteristic regulator to a different-family plant, the delivery medium comprising a partial structure of a plant belonging to the family Solanaceae, Brassicaceae, Lamiaceae, or Orobanchaceae.

2. The delivery medium according to claim 1, which is a delivery medium for delivery to a different-order plant.

3. The delivery medium according to claim 1 or 2, wherein the partial structure is a partial structure of a plant belonging to the family Solanaceae.

4. The delivery medium according to any one of claims 1 to 3, wherein the partial structure is a partial structure of a plant belonging to the genus *Nicotiana.*

5. The delivery medium according to any one of claims 1 to 4, wherein the characteristic regulator is at least one member selected from the group consisting of RNA and proteins.

6. The delivery medium according to any one of claims 1 to 5, wherein the delivery medium comprises a plant virus for producing a characteristic regulator.

7. A partial structure comprising the delivery medium according to any one of claims 1 to 6 grafted with a different-family plant directly or through another plant.

8. A plant body containing the partial structure according to claim 7.

9. A plant body characteristic-regulating method comprising delivering a plant virus for producing a characteristic regulator to the plant body according to claim 8 through the delivery medium according to any one of claims 1 to 6 contained in the plant body.

10. A method for producing a characteristic-regulated plant body, the method comprising delivering a plant virus for producing a characteristic regulator to the plant body according to claim 8 through the delivery medium according to any one of claims 1 to 6 contained in the plant body.

11. The characteristic-regulating method according to claim 9, the method comprising grafting the delivery medium according to claim 6 with a different-family plant directly or through another plant.

12. The production method according to claim 10, the method comprising grafting the delivery medium according to claim 6 with a different-family plant directly or through another plant.

13. A seed of a characteristic-regulated plant body obtained from the plant body according to claim 8 or an offspring plant body of the plant body according to claim 8.

14. A seed group of a characteristic-regulated plant body obtained from the plant body according to claim 8 or an offspring plant body of the plant body according to claim 8.

15. A method for producing a seed of a characteristic-regulated plant body, the method comprising obtaining a seed from the plant body according to claim 8 or an offspring plant body of the plant body according to claim 8.


### Sequence Listings

SEQUENCE LISTING

<110>  National University Corporation Nagoya University

<120>  Virus vector delivery medium

<130>  P17-138WO

<150>  JP 2016-164729
<151>  2016-08-25

<160>  15

<170>  PatentIn version 3.5

<210>  1
<211>  18
<212>  DNA
<213>  Artificial Sequence

<220>
<223>  CPTALL-3

<400>  1
gactgaccat tttagccg                                                    18


<210>  2
<211>  20
<212>  DNA
<213>  Artificial Sequence

<220>
<223>  CPTALL-5

<400>  2
yasytttdrg gttcaattcc                                                  20


<210>  3
<211>  25
<212>  DNA
<213>  Artificial Sequence

<220>
<223>  AtACT2_F primer

<400>  3
ttcagatgcc cagaagtctt gttcc                                            25


<210>  4
<211>  22
<212>  DNA
<213>  Artificial Sequence

<220>
<223>  AtACT2_R primer

<400>  4
ctggacctgc ctcatcatac tc                                               22

<210>  5
<211>  23
<212>  DNA
<213>  Artificial Sequence

<220>
<223>  NbACT_F primer

<400>  5
cctgaggtcc ttttccaacc atc                                              23


<210>  6
<211>  24
<212>  DNA
<213>  Artificial Sequence

<220>
<223>  NbACT_R primer

<400>  6
gcctttgcaa tccacatctg ttgg                                             24


<210>  7
<211>  23
<212>  DNA
<213>  Artificial Sequence

<220>
<223>  ALSV_F

<400>  7
gagtggtcat gctaccattc gag                                              23


<210>  8
<211>  25
<212>  DNA
<213>  Artificial Sequence

<220>
<223>  ALSV_R

<400>  8
caggtgaact ttccccaaaa gcttg                                            25


<210>  9
<211>  24
<212>  DNA
<213>  Artificial Sequence

<220>
<223>  TRV2_F

<400>  9
ctgggagatg atacgctgtt tgag                                             24


<210>  10
<211>  28
<212>  DNA

<213>  Artificial Sequence

<220>
<223>  TRV2_R

<400>  10
gaacctaaaa cttcagacac ggatctac                                          28


<210>  11
<211>  23
<212>  DNA
<213>  Artificial Sequence

<220>
<223>  BnaACT2_F

<400>  11
ctgtcccaat ctacgagggt ttc                                               23


<210>  12
<211>  20
<212>  DNA
<213>  Artificial Sequence

<220>
<223>  BnaACT2_R

<400>  12
gctctgctgt cgtggtgaac                                                   20


<210>  13
<211>  201
<212>  DNA
<213>  Arabidopsis thaliana

<400>  13
catggttcca agatggcatt cttggatggt aatcctccgg aaaggctttg tatgccagta       60

gtggatcata ttcgatcact aggtggggaa gtgcaactta attctaggat aaagaaaatt      120

gagctcaatg acgatggcac ggttaagagt ttcttactca ctaatggaag cactgtcgaa      180

ggagacgctt atgtgtttgc c                                                201


<210>  14
<211>  26
<212>  DNA
<213>  Artificial Sequence

<220>
<223>  AtPDS_F

<400>  14
gatagctttg aaccggtttc ttcagg                                            26


<210>  15
<211>  27
<212>  DNA

<213>  Artificial Sequence

<220>
<223>  AtPDS_R

<400>  15
gaagtacggt atttctttcc agggatc                                          27